# EUROPÄISCHE PATENTSCHRIFT

(11) **EP 1 490 691 B1**
(45) Veröffentlichungstag und Bekanntmachung des Hinweises auf die Patenterteilung: **21.01.2009**
(21) Anmeldenummer: 03720372.6
(22) Anmeldetag: 27.03.2003
(51) Int. Cl.: G01N 33/58, G01N 33/543, C12Q 1/68

(54) **LUMINESZIERENDE, SPHÄRISCHE, NICHT AUTOFLUORESZIERENDE SILICAGEL-PARTIKEL MIT VERÄNDERBAREN EMISSIONSINTENSITÄTEN UND -FREQUENZEN**
LUMINESCENT, SPHEROID, NON-AUTOFLUORESCENT SILICA GEL PARTICLES HAVING VARIABLE EMISSION INTENSITIES AND FREQUENCIES
PARTICULES DE SILICAGEL LUMINESCENTES, SPHERIQUES ET NON AUTOFLUORESCENTES A INTENSITES ET FREQUENCES D'EMISSION VARIABLES

(30) Priorität: 30.03.2002 DE 10214019
(43) Veröffentlichungstag der Anmeldung: 29.12.2004
(73) Patentinhaber: MagnaMedics GmbH, 52066 Aachen (DE)
(72) Erfinder: Müller-Schulte, Detlef P., 52066 Aachen (DE)
(74) Vertreter: Mann, Volker
(86) Internationale Anmeldenummer: PCT/EP2003/003163
(87) Internationale Veröffentlichungsnummer: WO 2003/083481

(56) Entgegenhaltungen:
- DE-A- 2 426 919
- US-A- 5 585 640
- US-A- 5 789 162
- CHANG SONG-YUAN ET AL: "Preparation and Properties of Tailored Morphology, Monodisperse Collodial Silica-Cadmium Sulfide Nanocomposites" J. AM. CHEM. SOC., Bd. 116, Nr. 15, 1994, Seiten 6739-6744, XP001154849
- CORREA-DUARTE MA ET AL: "Stabilization of CdS semiconductor nanoparticles against photodegradation by a silica coating procedure" CHEMICAL PHYSICS LETTERS, Bd. 286, 17. April 1998 (1998-04-17), Seite 497-501 XP002256994
- CHAN WARREN C W ET AL: "Luminescent quantum dots for multiplexed biological detection and imaging" CURRENT OPINION IN BIOTECHNOLOGY, Bd. 13, Nr. 1, Februar 2002 (2002-02), Seiten 40-46, XP002256995 ISSN: 0958-1669

## Beschreibung

Die vorliegende Erfindung betrifft lumineszierende, sphärische Mikro- und Nanopartikel mit veränderbaren Lumineszenzintensitäten, ein Verfahren zu ihrer Herstellung sowie deren Verwendung.

Als Lumineszenz wird im folgenden die Emission von Licht des gesamten Spektralbereichs durch Stoffe definiert, die zuvor durch Energieabsorption angeregt wurden. Die Fluoreszenz und Phosphoreszenz fallen gleichfalls unter diesen Begriff.

Lumineszierende Stoffe werden heute in Form fluoreszierender oder phosphoreszierender Substanzen im gesamten Bereich der biochemischen und medizinischen Analytik bzw. Diagnostik routinemäßig zum Nachweis von Analyten wie Proteine, Peptide, Toxine oder Nukleinsäuren sowie als Marker zur Sichtbarmachung von Zellen, Zellkompartimenten oder zur Erfassung biologischer Prozesse eingesetzt.

Ein besonders interessante und zukunftsträchtige Nutzung der Lumineszenz-Methodik ergibt sich bei der so genannten Array-Technik zum Nachweis von Biomolekülen. Unter Array-Technik versteht man im Bereich der heutigen Bioanalytik und Diagnostik flache Glas- oder Polymerträger bzw, Substrate - auch als Biochips bezeichnet -, auf denen in rasterartiger Anordnung eine Vielzahl von Biomolekülsonden (Nukleinsäuren, Proteine oder Peptide) bekannter Struktur aufgebracht sind. Durch Inkubation Lumineszenz -markierter Protein- bzw. Nukleinsäureproben, die zuvor z. B, aus Gewebe oder Zellen gewonnen wurden, mit dem Biochip kommt es an den Stellen auf seiner Oberfläche zu einer spezifischen Bindung, die ein zur Probe komplementäre chemisch-physikalische Struktur aufweist. Aufgrund der Lumineszenzmarkierung der Probe resultiert an der Bindungsstelle eine detektierbare Lumineszenz, die eine direkte Information über die chemische und physikalische Struktur der Probe liefert. Infolge der hohen Anzahl der auf dem Chip immobilisierbaren Sonden stellt die Array-Technologie ein wertvolles Werkzeug auch für die Austestung von Molekülbibliotheken im Zuge der Pharmaka-Entwicklung dar.

Da die Qualität und die Anwendbarkeit der Chip-Technologie unmittelbar von der Nachweisempfindlichkeit sowie der spektralen Spezifität der Lumineszenzmarkers abhängt, wurden in der Vergangenheit vielfältige Entwicklungen in Richtung neuer, verbesserter Lumineszenzsysteme unternommen, um so die Nachweisgrenzen für den Bioassay entscheidend zu reduzieren.

Die Entwicklung lumineszierender Substanzen betrifft vorwiegend zwei Stoffklassen. Bei der einen handelt es sich um lumineszierende Moleküle, deren bekannteste Vertreter Fluorescein, Rhodamin, Phycoerythrin, Cumarin, Cy3, Cy5, TAMRA, ROX, Oregon Green, Ethidiumbromid, Texas Red und Dabcyl sind (bei diesen Namen handelt es sich durchweg um Handelsnamen). Die andere Verbindungsklasse stellen die vornehmlich aus den Gruppen IIB und VIA, IIIA und VA oder IVA des Periodensystems gebildeten Halbleiter - Nanokristalle dar, deren bekannteste Vertreter CdS, CdSe, CdTe, ZnS und ZnSe sind. Ein besonderes Merkmal dieser Halbleiter - Nanokristalle ist zum einen die hohe Quantenausbeute, zum anderen nelen sie im Gegensatz zu den herkömmlichen Lumineszenzfarbstoffen praktisch nicht zum Ausbleichen. Ein weiterer bemerkenswerter Punkt, der die in der Literatur als ""Qantum Dots"" bezeichneten Verbindungen zu einer interessanten Alternative zu den herkömmlichen Fluoreszenzfarbstoffen hat werden lassen, ist die Umstand, dass das Absorptions- und Emissionsverhalten der quantumdots grössenabhängig ist.

D.h. konkret; mit abnehmender Teilchengrösse verschiebt sich das Emissionsspektrum in den kürzerwelligen Bereich und umgekehrt.

Für die Entwicklung eines Bioassays unter Nutzung des Lumineszenzeffektes eröffnet das zusätzlich die Möglichkeit, unterschiediiche Lumineszenz-Markierungen nicht allein über die Substanzauswahl, sonder auch über die Teilchengrösse vorzunehmen. Dles bietet eine hervorragende Basis zur optischen Kodierung von Biomolekülen im Rahmen der Bioarray-Entwlcklung.

Die Verwendung von Fluoreszenzmarkern in der Bioanalytik ist vielfältig in der Literatur beschrieben.

US Patent 4,777,123 beschreibt ein Immunoasay-Prinzip, das die Verwendung von mit zwei unterschiedlichen Fluorophoren markierten Rezeptoren offenbart, wobei der Energietransfer vom angeregten ersten Fluorophor auf den zweiten Fluorophor durch die Anwesenheit des zu detektierenden Liganden reduziert wird.

Gegenstand des US Patentes 5,324,633 sind Bioarray-Methoden zur Erfassung von an Biopolymeren gebundene Fluoreszenz-markierte Rezeptoren mit Hilfe eines Photonenzählers oder der konfokalen Mikroskopie.

Xanthen-Fluoreszenz-Farbstoffe mit einer Anregungsfrequenz von 450-650 nm zur Zell-Detektion mit Hilfe markierter Antikörper sind im US Patent 5,066,580 ausgeführt.

In den US Patenten 3,998,943, 3,996,345, 4,174,384, 4,199,559 und 4,261,968 werden Liganden-Rezeptorassays offenbart, deren Grundprinzip in der Markierung eines Rezeptors und/oder Liganden mit einem Fluoreszenzfarbstoff besteht, dessen Emissionsverhalten in Abhängigkeit von der Liganden-Rezeptorbindung detektiert wird.

Im US Patent 5,319,209 sind Fluoreszenz-Sonden u. a. in Form von Benzofuranisophthalat offenbart, die zur Messung von lonenkonzentrationen in Zellen geeignet sind.

Die Herstellung von Halbleiter-Nanokristallen bzw. "Qantum Dots" mit den entsprechenden optischen Eigenschaften ist allgemeiner Stand der Technik und in der Literatur divers beschrieben: Kortan et al., J. Am. Chem. Soc. Vol 112, 1327, 1990 ; Colvin et al., Nature, Vol, 370, 354, 1994 ; Murray et al., J. Am. Chem. Soc. Vol. 115, 8706, 1993 ; Hirai et al., J. Phys. Chem. B, Vol. 103, 4228, 1999 ; Dabbousi et al., J. Phys. Chem. B, Vol. 101,9463, 1997 ; Hines et al., J. Phys. Chem. Vol. 100, 468, 1996 ; Danek et al., Chem. Mater. Vol. 8, 173, 1998, Steigerwald et al., J. Am. Chem. Soc, (1988) 110 : 3046-3050 und Lianos et al., Chem. Phys. Lett. Vol. 125, 299, 1988.

Für die Anwendung lumineszierender Substanzen in der Bioanalytik ist die eigentliche Synthese jedoch nicht allein ausschlaggebend, mitentscheidend ist parallel die Möglichkeit, die Lumineszenz-Marker an die entsprechenden Biosubstanzen kovalent zu binden. Bei Lumineszenzfarbstoffen werden funktionelle Gruppen vorwiegend in Form von N-Hydroxysuccinimidyl-Estern oder Isothiocyanat-Funktionen eingeführt, die mit den Aminogruppen der Biomoleküle eine Bindung eingehen.

Die Funktionalisierung der "Qantum Dots" geschieht dagegen grundsätzlich nach zwei Verfahrensweisen: zum einen werden die Oberflächen mit funktionellen Mercaptoverbindungen wie beispielsweise Mercaptopropionsäure, Dihydroliponsäure, Mercaptoessigsäure, Mercaptosilanen umgesetzt, die dann vorzugsweise über die Carboxylfunktion an die entsprechenden Biomoleküle gekoppelt werden (Gerion et al.,J. Phys. Chem. Vol. 105,8861, 2001; Mattoussi et al., J. Am. Chem. Soc. Vol. 122, 12142, 2000 ; Chan et al., Science Vol 281, 2016, 1998 ; Mitchell et al., J. Am. Chem. Soc. Vol. 121, 8122, 1999) oder die "Qantum Dots" werden in eine Polymermatrix oder Dendrimere eingekapselt ("capping"). Lemon et al. (J. Am. Chem. Soc. Vol. 122, 12886, 2000), Sooklal et al. (Adv. Mater. Vol. 10, 1083, 1998) und Lakowicz et al. (J. Phys. Chem. Vol. 103, 7613, 1999) beschreiben die Einkapselung der Halbleiter-Nanokristalle mit Hilfe von Polyamidoamin-Dendrimeren. Einkapselungen unter Zuhilfenahme von Polystyrol-co-Vinylpyridin, Polystyrol, Silicagel oder Polylaurylmethacrylat werden von Zhao et al. (Chem. Mater. Vol. 14, 1418, 2002), Han et al. (Nature Biotech., Vol. 19, 631, 2001), Correa-Duarte et al. (Chem. Phys. Letters, Vol. 286, 497, 1998), Chang et al. (J. Am. Chem. Soc. Vol. 116, 6739, 1994) und Lee et al. (Adv. Mater., Vol. 12, 1102, 2000) beschrieben.

"Qantum Dots", die mit Mercaptokomponenten und Diaminocarboxylsäuren umhüllt sind sowie Halbleiter-Nanokristalle, die in der Gasphase mit Hilfe eines Sprühsystems hergestellt werden, sind Gegenstand der US Patente 6,114,038 und 5,906,670.

Die Herstellung von Halbleiter-Nanokrlstalle aus der Gruppe III-V und 11-VI, die zur Kopplung von Affinitäts-Liganden befähigt sind, gehen aus den US Patenten 6,207,397, 5,251,018, 5,751,018, 5,262,357 und 5,505,928 hervor,

Gegenstand der US Patente 6,326, 144 und 6,207, 229 sind beschichtete Halbleiter-Nanokristalle mit einer Affinitätsbindung zu biologischen Komponenten bzw. monodisperse, mit ZnS, ZnSe, CdS oder CdSe beschichtete lichtemittierende Nanokristalle.

US Patente 5,293,050 und 5,354,707 offenbaren lichtemitierende Halbleiterschichten aus Silicon für Schaltkreise, in denen die zweite Silicon-Schicht mindestens einen Quantum Dot enthält.

In US Patent 5,525,377 werden 20 bis 100 Å große, mit Polymethylmethacrylaten beschichtete Nanopartikel aus dotierten Halbleiter-Verbindungen für den Einsatz als Kathodenstrahlröhren oder Elektrolumineszenz Displays beschrieben.

Die Verwendung von Halbleiter-Nanokristallen in Form von ZnS, ZnSe, ZnTe, CdS, CdSe, CdTe; HgS, HgSe, HgTe, GaS, InAs, InP und InSb als Elektronen-Transportmedium zur Herstellung von Elektrolumineszenz - Geräten, die bei angelegter Spannung sichtbares Licht verschiedener Wellenlängen emittieren, gehen aus dem US Patent 5,537,000 hervor.

Mit Übergangsmetallen dotierte laserverstärkende Kristalle, bestehend aus Gruppe II und VI, sind Gegenstand des US Patentes 5,541,948.

Eine mit Lumineszenz - Nanokristallen dotierte Glasmatrix wird im US Patent 5,585,640 beschrieben. Zur Herstellung der dotierten vorgefertigten Glasformen werden Temperaturen über 1000 °C benötigt.

US Patent 5,770,299 offenbart Halbleiter-Nanokristalle bestehend aus CdS und/oder III-V Komponenten oder II-VI Halbleiter-Nanokristalle als Pigmente für Anstrichfarben.

Eine Festphasen-Synthese von Fluoreszenz - markierten Peptid- Bibliotheken auf kommerziellen Silicagel-Beads werden im US Patent 5,789,162 beschrieben.

Lumineszenz-Halbleiter-Nanokristall-Sonden für biologische Applikationen, die mit Silanderivaten beschichtet und Affinitäts-Moleküle zu binden befähigt sind, sind im US Patent 5,990, 479 ausgeführt.

US Patent 5,043,265 offenbart Fluoreszenz Partikel in Form von ZnS-Ag und Y₂0₂S-Eu als Label für Immunoglobuline und Polynukleotide,

Die Herstellung von Nanopartikeln aus Übergangsmetallen, Lanthaniden oder Actiniden in Gegenwart eines in einem inerten Gelpolymeren dispergierten, in Form von Nukleinsäure oder Proteinen vorliegende Kationenaustauscher, ist Gegenstand des US Patents 5,985,353. Durch Zugabe entsprechender Anionen werden die an den Austauschern gebundenen Kationen zu Nanopartikeln ausgefällt.

Phycobiliprotein-Marker, die den Grad der spektralen Überlappung zwischen der Anregungs- und Emissionsfrequenz reduzieren können, entsprechend einer deutlichen Stokes-Verschiebung, sind im US Patent 4,666,862 sowie von Ol et al. (J. Cell. Biol. Vol. 93, 891 1982) beschrieben.

Eine weitere Gruppe Licht emittierender Substanzen stellen die so genannten up-converting phosphors dar, deren Besonderheit in der Emission kürzerwelliger Strahlung gegenüber der Anregungstrahlung besteht, Substanzen dieser Art setzen sich weitgehend aus Verbindungen des Sauerstoffs und/oder Schwefels und/oder Fluors mit den Lanthanoiden oder Yttrium zusammen.

Für die Verwendung im Bioassay haben diese Substanzen den Vorteil, dass ihre Absorptions- und Emissionsfrequenzen nicht durch Autofluoreszenz der Trägersysteme wie Trägerpartikel oder Mikrotiterplatten gestört werden, ein Phänomen, das in der heutigen Bioanalytik bei der Verwendung von Trägersystemen jedweder Art ein nach wie vor ungelöstes Problem darstellt.

In den US Patenten 5,674,698 und 5,698,397 sind eine Reihe von Molekül-Sonden in Form dotierter up-converting phosphore wie z. B. Na-Yttriumfluorid,Na-Yttriumfluorid, Lanthanfluorid. Gadoliniumfluorid und Yttriumoxysulfide beschrieben, die mittels Laseranregung in biologischen sowie anderen Assays genutzt werden.

In Polyacrylat-Mikroträgern eingekapselte up-converting phosphor Partikel, die für die Anregungs- und Emissionsfrequenzen transparent sind und über funktionelle Gruppen Protein-Sonden kovalent binden können, sind im US Patent 5,132,242 ausgeführt.

Up-converting phosphore für die Detektion von Nukleinsäuresequenzen bzw. für den Immunoassay sind von Corstjens et al. (Clin. Chem. Vol. 47,1885, 2001) und Niedbala et al. (Anal. Biochem. Vol. 293,22, 2001), Silanbeschichtete upconverting phosphore von Hampl et al. (Anal. Biochem. Vol. 288, 176, 2001) beschrieben.

Lumineszierende Metallporphyrine, die zur Detektion von biologischen Substanzen verwendet werden können, sind Gegenstand des US Patentes 6,004,530.

Die aus dem Stand der Technik bekannten Lumineszenzmarker in Form von Einzelmolekülen oder Nanokristallen haben den Nachteil, dass ihre Verwendung speziell für den Bioassay aufwendige Präparationen sowohl in Bezug auf den zeitlichen als auch den verfahrenstechnischen Aufwand erfordern. So sind in der Regel Synthese- bzw. Präparationszeiten von mehreren Stunden bis hin zu mehreren Tagen (vgl. US Patent 5,132,242) in Verbindung mit aufwendigem verfahrenstechnischem Aufwand notwendig; Arbeiten unter Stickstoff-Inertgasatmosphäre, Destiliationen, Arbeiten in organischen Medien. Darüber hinaus weisen die Produkte, sofern es sich um organische Polymere handelt, eine ausgeprägte Autofluoreszenz auf, vgl. Han et al., als Referenz oben aufgeführt.

Die gravierendste Einschränkung der bekannten Lumineszenz Systeme ist, dass in der Regel jeweils nur ein oder sehr wenige Lumineszenz Partikel (Halbleiter-Nanokristalle oder up-converting phosphor Kristall) pro zu markierendem Biomolekül gebunden werden. Diese Beschränkung reduziert konsequenterweise die Nachweisempfindlichkeit des Bioassays nachhaltig. Ähnliches gilt auch für Lumineszenzmoleküle, von denen auch nur eine sehr geringe Zahl pro Biomolekül gebunden werden können, so dass das von einem markierten Biömolekül ausgehende Signal schwierig ist zu detektieren.

Hinzu kommt, dass durch die Kopplung der lumineszierenden Substanz an das Biomolekül (z. B. Antikörper) dieses inaktiviert werden kann. Bei den alternativ dazu entwickelten Lumineszenz - markierten Mikro- oder Nanopartikeln handelt es sich jedoch durchweg um auf der Oberfläche der Partikel gebundene Lumineszenzmarker, deren Herstellung, Anwendung und Detektion schwierig ist; vgl.: Fornusek und Vetvicka, "Polymeric Microspheres as Diagnostic Tools for Cell Surface Marker Tracing," in CRC Critical Reviews in Therapeutic Drug Carrier Systems, 2, pp. 137-174,1 986. Der Artikel enthält eine kritische Übersicht über diese Technik, des Weiteren, hier als Referenz angegeben: Kaplan et al. (Biochimica et Biophysica Acta, Vol. 728, 112, 1983) sowie die US Patente 3,853,987, 4,035,316, 4,105,598, 4,108,972, 4,224,198 und 4,326,008.

DE 24 26 919 A1 betrifft Baumaterial mit photolumineszierenden Eigenschaften, das einerseits die für ein Baumaterial erforderliche Festigkeit aufweist, andererseits nach Auslöschen eines Ausgangslichtes im Volumen Iuminesziert, Als photolumineszierendes Material wird Zinksulfid eingesetzt. Dem Beton kann ein lichtdurchlässiges Material, zum Beispiel Silicagel, zugesetzt werden, das in Form eines Koiloids vorliegen kann.

In Chemical Physics Letters, 286 (1998), Seiten 497 bis 501 wird ein zementhaltiges Baumaterial, das Zinksulfid und gegebenenfalls ein kolloidales Silicagel enthalten kann, beschrieben. Dagegen handelt es sich gemäß der vorliegenden Erfindung um sphärische (also kugelförmige) Silicagel-Partikel, deren Matrix Iumineszierende Substanzen enthalten kann.

In dem Dokument wird die Verhinderung des Abbaus von Cadmiumpartikel durch Lichteinwirkung durch Beschichtung mit Silicagel.

J. Am. Chem. Soc. 1994, 116, Seiten 6739 bis 6744 betrifft CdS Quantum Dots, die in monodisperse Silicagelkollodide eingelagert sind. Die Herstellung erfolgt in w/o Microemulsionen. Das poröse Material kann als Katalysator verwendet werden.

In dem International J. of Pharmaceutics 200 (2000) 223 bis 229 werden kugelförmige Silicagel-Partikel beschrieben, die durch Sprühtrocknung hergestellt werden. Die Silicagel-Matrix enthält keine lumineszierenden Substanzen, sondern Toremifene-Citrat.

Aufgabe der vorliegenden Erfindung ist es, die Nachteile der aus dem Stand der Technik bekannten Lumineszenz-Trägersysteme zu umgehen und lichtdurchlässige, nicht autofluoreszierende mikro- und submikropartikuläre Teilchen mit adaptierbarer Porosität bereitzustellen, die multiplexe lumineszierende Substanzen enthalten. Durch die Möglichkeit der Einkapselung einer Vielzahl lumineszierender Agenzien sowohl in Form von Molekülen als auch Nanopartikeln bzw, Kolloiden kann überraschenderweise die Nachweisempfindlichkeit für Biomoleküle im Rahmen der Bioanalytik und Diagnostik so gesteigert werden, dass die Möglichkeit zum Nachweis einzelner Moleküle möglich wird. Des Weiteren kann durch die Einkapselung mehrerer Lumineszenz-Substanzen mit unterschiedlichem Emissionsverhalten eine Vielzahl optischer Kodierungen der Biomoleküle vorgenommen werden.

Gegenstand der Erfindung sind neue sphärische, lumineszierende Silicagel-Partikel, die in einer transparenten Silicagel-Matrix eine oder mehrere lumineszierende Substanzen enthalten, wobei die Silicagel-Matrix erhältlich ist, indem eine Mischung bestehend aus einer verdünnten Säure und Alkoxysilanen zu einem klaren Silica-Sol kondensiert wird; das klare Silica-Sol mit einem oder mehreren lumineszierenden Substanzen homogen vermischt wird; die Sol-Lumineszenz-Substanz-Mischung in einer mit Wasser nicht mischbaren organischen Phase dispergiert und die Sol-Lumineszenz-Substanz-Mischung während oder nach dem Dispergiervorgang durch Zugabe einer Base vernetzt wird und wobei die Silicagel-Matrix funktionelle Gruppen aufweist, die mit Biomolekülen koppelbar sind.

Die Erfindung wird bevorzugt durch ein inverses Suspensions-Verfahren gelöst, indem eine Mischung, bestehend aus einem Silicagel-Sol und der lumineszierenden Substanz, in einer mit Wasser nicht mischbaren organischen Phase dispergiert und anschließend polykondensiert wird.

Das bevorzugte Verfahren zur Herstellung der erfindungsgemäßen sphärischen, lumineszierender Silicagel-Partikel, ist dadurch gekennzeichnet, dass eine Mischung bestehend aus einer verdünnten Säure und Alkoxysilanen zu einem klaren Silica-Sol kondensiert wird; das klare Silica-Sol mit einem oder mehreren lumineszierenden Substanzen homogen vermischt wird; die Sol-Lumineszenz-Substanz-Mischung in einer mit Wasser nicht mischbaren organischen Phase dispergiert und die Sol-Lumineszenz-Substanz-Mischung während oder nach dem Dispergiervorgang durch Zugabe einer Base vernetzt wird.

Dadurch werden dreidimensional vernetzte, perlförmige Polymerträger gebildet, die die Lumineszenz-Substanz eingekapselt enthält.

Silica-Sole werden nach den aus dem Stand der Technik allgemein bekannten Verfahren durch Hydrolyse von Alkoxysilanen mit Hilfe verdünnter Mineralsäuren (Dave et al. in: Immobilized Biomolecules in Analysis, Hrsg. Cass und Ligler, Oxford University Press, 1998; WO 02/09125A1) hergestellt.

Als Alkoxysilane können Kieselsäureorthoester aliphatischer Alkohole eingesetzt werden, wobei bevorzugt Methyl-, Äthyl- oder Propylester einzeln oder als Mischungen verwendet werden. Zu dem Silica-Sol werden im zweiten Schritt die entsprechenden Lumineszenz-Substanzen zugegeben. Um das heterogene Sol-Lumineszenz-Substanz Gemisch homogener durchmischen zu können, wird die Reaktion in einem Ultraschallbad oder unter Zuhilfenahme einer Ultraschallsonotrode durchgeführt. Der Beschallungsvorgang dauert in der Regel eine halbe Minute. Die so gewonnene kolloiddisperse Mischung wird anschließend in einem organischen, nicht mit Wasser mischbaren Lösungsmittel in der Regel unter Rühren dispergiert. Dabei werden perlförmige Sol-Tröpfchen gebildet, die durch anschließende Zugabe einer verdünnten Base zu dreidimensional vernetzten Silicagelen polykondensieren.

Die Polaritätseigensohaften des Sols und der organischen Dispersionsphase sind so gewählt, dass zum einen eine stabile Suspension gebildet wird, zum anderen die zugesetzte Lumineszenz-Substanz eine hohe Affinität zu der Sol-Phase besitzt, so dass eine klare Trennung zwischen Sol- und organischer Phase stattfindet und demzufolge sich die zugesetzte lumineszierende Verbindung ausschließlich in der Sol- Phase homogen verteilt.

Der gesamte Herstellungsprozess, einschließlich der Präparation der Silicagel-Sole, erfordert je nach Ansatz einen Zeitaufwand von 20 bis 50 Minuten.

Ausgangspunkt der Synthese der Lumineszenz-Partikel sind Silica-Sole, die nach bekannten Verfahren durch Hydrolyse geeigneter Alkoxysilane in saurem, wässrigem Milieu gebildet werden. Für das Verfahren und Produkt werden bevorzugt solche Silane eingesetzt, die einen C₁ bis C₃ Esterrest besitzen. Dies ermöglicht überraschenderweise zusammen mit einer definierten Konzentration der Silane die Herstellung voilkommen transparenter Partikel. Die Konzentrationen der Alkoxysilane im Anfangsansatz liegen zwischen 40 und 90 Vol%, vorzugsweise zwischen 65 und 80 Vol%. Durch Beschallen der Silane in Gegenwart einer wässrigen Säurelösung, deren Volumenanteil im Silanansatz in der Regel zwischen 10 und 40 Vol% und deren Konzentration in der Regel zwischen 0,01 und 0,5Mol/Liter beträgt, werden klare Sole gebildet. Die Beschallungszeiten dauern je nach Säurekonzentration 5 bis 20 Minuten. Die so gewonnenen Silica-Sole lassen sich mehrere Tage bei Tiefkühltemperaturen (ca.-18 °C) aufbewahren oder können wahlweise sofort weiterverarbeitet werden,

Ein weiteres Merkmal der erfindungsgemäßen Silicagel-Technologie besteht darin, die Porosität der gewonnenen Partikel in weitem Rahmen zu variieren, ein Parameter, den kein anderes aus dem Stand der Technik bekanntes Verfahren in der Weise bietet.

Die Porosität von partikulären Trägermedien spielt bei sämtlichen Bioassays oder Separationsprozessen insofern eine entscheidende Rolle, als die spezifische Nachweisbarkeit des Analyten von dessen Anbindung an die auf dem Träger immobilisierten Liganden- bzw. Rezeptoren abhängt. Das Ausmaß und die Qualität dieses Bindungsprozesses wird direkt von der zugänglichen Oberfläche des Trägers beeinflusst, Letztere wird unmittelbar von der Porosität des Trägers bestimmt.

Diese kann überraschenderweise mit Hilfe der erfindungsgemässen Produkte und Verfahren über den Zusatz bestimmter Stoffe zu dem Sol eingestellt werden. Diese allgemein als Porogene bezeichneten Substanzen werden dem Sol vor der Dispersion zugegeben. Ais Zusätze kommen bevorzugt solche Stoffe In Frage, die das Absorptions-Emissions-Verhalten der Träger nicht beeinträchtigen. Die Erfindung nicht einschränkende Beispiele hierfür sind: Polyethylenglykol, Polyvinylalkohol, Polyacrylsäure, Polyaminosäuren, Polysaccharide, Proteine oder Polyvinylpyrrolidon. Sie werden während des Dispersions-und Vernetzungsprozesses in die sphärischen Partikel eingekapselt. Der Volumenanteil der organischen Polymerlösungen, die durchweg als 1 bis 10% ige wässrige Lösungen vorliegen, liegt zwischen 1 und 20 % bezogen auf die Sol-Phase.

Ein weiterer Parameter zur Einstellung der Porosität ergibt sich aus der Auswahl der Zusammensetzung der verschiedenen di- tri- oder tetrasubstituierten Silankomponenten. So weisen Gele aus rein tetrasubstituierten Estern grundsätzlich eine geringere Porosität auf als Sole bzw. Gele, die unter Zusatz von di- oder tri-substituierter Esterverbindungen hergestellt wurden. Durch gezieltes Zumlschen der di- oder trifunktionellen Esterverbindungen lassen sich so Porenweiten im Bereich von 50-200 nm realisieren. Die Konzentration der di- und trifunktionellen Silane betragen in der Regel 1-10, vorzugsweise 1 bis 5 Vol%, bezogen auf die Gesamtsilankonzentration.

Ein weiterer, wesentlicher Aspekt der erfindungsgemässen Produkte und Verfahren besteht darin, die Teilchengrösse sowohl über die Art und Weise des mechanischen Rührens als auch über die Viskosität des Silica-Sols zu steuern. Bekannterweise werden die Teilchengrössen bei einer Suspensionspolymerisation über die Rührgeschwindigkeit eingestellt, derart, dass grundsätzlich mit zunehmender Rührgeschwindigkeit die Teilchengrösse abnimmt. Der Zusammenhang dieser Parameter trifft auch bei der vorliegenden Erfindung zu, d. h., Rührgeschwindigkeiten > 1000 U/Min. führen allgemein zu Teilchengrössen < 50 im, solche von > 5000 U/Min. durchweg zu Teilchengrössen < 10 im. Zur Erzielung noch feinerer Partikel im submikrometer Bereich sind Dispergierwerkzeuge, die z. B. nach dem Rotor-Stator-Prinzip arbeiten und eine Rotorgeschwindigkeit von > 10,000 U/Min. aufweisen (z. B. Uitra-Turrax®), erforderlich. Auch durch Behandlung mit Ultraschall lassen sich Teilchengrössen im Bereich von 0,5 bis 10 im realisieren.

Neben der rein mechanischen Einflussgrösse konnte gezeigt werden, dass auch die Viskosität des Sols einen entscheidenden Einfluss auf die Partikelgrößen hat. Sinkende Viskosität des Sols fährt in der Regel zu einer analogen Verringerung der Teilchengrössen und umgekehrt: steigende Viskositäten führen allgemein zu einer Teilchenvergrößerung. Die zur Herstellung der erfindungsgemässen Produkte mit den gewünschten Partikelgrössen von 0,5 bis 50 im erforderlichen Viskositäten liegen im Bereich von 5 bis 300 cp.

Nach der Präparation des Sols mit den gewünschten Eigenschaften wird im darauf folgenden Schritt der entsprechende Lumineszenzmarker zugesetzt und mit dem Sol innig vermischt, vorzugsweise unter Zuhilfenahme einer Ultraschall- Behandlung. Als lumineszierende Substanz kommen grundsätzlich solche Verbindungen in Frage, die nach Absorption von Strahlung einer bestimmten Wellenlänge zur Emission von Photonen einer von der Absorptionsfrequenz unterschiedlichen Frequenz befähigt ist und mit dem Silicagel-Sol eine homogene Mischung einzugehen in der Lage ist.

Substanzen dieser Art sind in der als Referenz aufgeführten Literatur vielfältig beschrieben. Die das erfindungsgemäße Produkt nicht einschränkende Beispiele für lumineszierende Moleküle bzw. Marker sind: Fluorescein, Rhodamin, Coumarin, Dansylchlorid, Ethidiumbromid, Texas Red, Phycoerythrin, Oregon Green oder Cascade Blue weiterhin die unter der Handelsbezeichnung BODIPY, SYBR Green, TOTO-1 oder YOYO-1 bekannten Verbindungen sowie die Derivate dieser Produkte.

Als Halbleiter-Nanokristalle kommen Verbindungen aus der Reihe II-VI wie z. B. MgS, MgSe, MgTe, CaS, CaSe, CaTe, SrS, SrSe, SrTe, BaS, BaSe, BaTe, ZnS, ZnSe, ZnTe, CdS, CdSe, CdTe, HgS, HgSe oder HgTe sowie Nanokristalle der Gruppe III-V wie GaAs, InGaAs, InP oder InAs in Frage. Des Weiteren sind auch Halbleiter aus der Gruppe IVA wie z. B. Germanium geeignet.

Geeignete Beispiele für die up-converting phosphore sind Verbindungen aus Seltenen Erden oder Elementen der Gruppe IIIB wie: Na-Yttriumfluorid, Lanthanfluorid, Lanthanoxysulfid, Yttriumoxysulfid, Yttriumfluorid, Yttriumgallat, Gadoliniumfluorld, Barium-Yttriumfluoride, Gadoliniumoxysulfid sowie mit Aktivatorpaaren wie Ytterbium/Erbium, Ytterbium/ Thulium oder Ytterbium/Holmium dotierte Verbindungen des obigen Typs. Weiterhin als up-converting phosphore sind Chelat-Verbindungen aus Erbium, Neodym, Thulium, Holmium und Praseodym geeignet.

In analoger Weise lassen sich auch lumineszierende Proteine wie Rhodopsin, das grün-fluoreszierende Protein ("green fluorescent protein", GFP) sowie Metallporphyrine als Lumineszenz-Substanz einsetzen.

Die Konzentration der zugesetzten lumineszierenden Substanzen kann in weitem Rahmen variiert werden, je nach Erfordernis, die an die betreffende Bloanalytik oder Diagnostik gestellt ist. In der Regel reichen Konzentrationen von 1-10 Gew% der Markersubstanz aus, um eine deutliche Lumineszenz zu erzielen. Diese Konzentrationen übertreffen die bei den herkömmlichen Assays pro Analyt einsetzbaren Lumineszenzmarker-Konzentrationen um mehrere Zehnerpotenzen.

Aufgrund der hervorragenden Mischbarkeit der Silica-Sole mit den verschiedensten Substanzen, hat sich überraschenderweise die Möglichkeit ergeben, zusätzlich zu den Lumineszenz-Substanzen simultan auch magnetische Verbindungen in Form magnetischer Kollolde bzw. Nanopartikel mit einzukapsein. Dies ermöglicht eine völlig neuartige

Eigenschaftskombination, die die parallele Nutzung des Lumineszenzeffektes und der magnetischen Abtrennung eröffnet.

Hieraus ergeben sich völlig neue Perspektiven für die Entwicklung hocheffizienter Bioarrays, mit denen eine sehr hohe Zahl von Siomolekülen ("Molekül-Bibliothek") ausgetestet werden kann, die den bisherigen, ausschließlich auf Polymer- oder Glassubstraten durchgeführten Arrays verschlossen bleibt.

Als magnetische Substanzen kommen durchweg die bekannten ferro-, ferri- oder superparamagnetischen Substanzen sowie Ferrofluide in Frage, die in der einschlägigen Literatur vielfach beschrieben sind ; Br. Patent 1 439 031, Shinkai et al., Biocatalysis Vol 5, 61, 1991, Kondo et al., Appl. Microbiol. Biotechn. Vol, 41, 99, 1994.

Die Konzentrationen der Magnetkolloide werden durchweg so gewählt, dass die Transparenz der Partikel im Hinblick auf das Absorptions- und Emissionsverhalten der Lumineszenzmarker grundsätzlich nicht beeinträchtigt ist. Sie liegen in der Regel zwischen 10 und 30 Gew% bezogen auf die Silicagel-Partikel,

Im dritten Verfahrenschritt werden die Silicagel-Lumineszenzmarker-Mischungen in einer organischen Phase unter Rühren dispergiert. Als Dispergiermittel sind solche Lösungsmittel geeignet, die mit Wasser nichtmischbar sind und eine stabile Dispersion des Silica-Sols erlauben,

Dispersionsmittel dieser Art sind aus dem Stand der Technik allgemein bekannt und in WO 02/09125 sowie von Laane et al. ("Blocatalysis in Organic Media 55, Laane et al. Hrsg., Elsevier, Amsterdam, pp 65, 1987) beschrieben. Lösemittel, die diese Eigenschaften erfüllen, sind z. B. Hexan, Trichlorethylen, Petroläther, Toluol, Chloroform, 1,1,1-Trichloräthan, Tetrachlorkohlenstoff, Heptan, Auch Mischungen der obigen Lösungsmittel die eine Dichte von ca. 1 g/cm³ aufweisen, eignen sich gut zum Dispergleren. Die Volumenverhältnisse organische Phase zu Hydrosol betragen in der Regel 8:1 bis 30;1.

Im Hinblick auf eine engere Crößenverteilung der Partikel und auf ein besseres Dispergierverhalten können der organischen Phase eine oder mehrere oberflächenaktive Substanzen in Form von Tensiden, Dispersionsstabilisatoren oder Emulgatoren zugesetzt werden. Beispiele hierfür sind : Propylenoxid-Äthylenoxid-Blockcopolymere, Polyhydroxyfettsäure- Polyäthylenglykol-Blockcopolymere, Polyäthylenglykol-Ätherderivate, Sorbitan-Fettsäureester, Blockcopolymere aus Rizinusöl-Derivaten, Polyäthylenglykol-Castoröl-Derivate, Polyoxyäthylen-Sorbitan-Fettsäureester, Alkylphenylpolyäthylenglykol-Derivate, Polyglycerinester, modifizierte Polyester, Polyoxypropylen-Äthylendiamin-Blockcopolymere, Polyäthylenglykole, Polyoxyäthylen-, Polyoxyäthylen-Alkohol-Derivate, Substanzen dieser Art sind im Handel u. a. unter der Handelsbezeichnung: Renex®, Estol®, Prisorine®, Hypermer®, Pluronic®, Tween®, Eumulgin®, Pripol®, Tetronic®, Brij®, Lameform®, Arlacel®, Span®, Dehymuls®, Synperonic® oder Triton® bekannt. Die für die Herstellung der Partikel relevanten Tensid-Konzentrationen liegen zwischen 0,1 und 15%, vorzugsweise zwischen 0,5 und 6 Vol- bzw. Gew%.

Im letzten Verfahrensschritt werden die Sole während des Dispergiervorgangs mit einer verdünnten Base versetzt, wodurch erstere zu einem festen Gel polykondensieren. Die Fixierung der Sole zu den gewünschten Gel-Partikeln geschieht in der Regel innerhalb weniger Sekunden (3 bis 20 Sekunden). Dementsprechend erfordert der mechanische Dispergiervorgang auch nur wenige Sekunden. Der Gelbildungs- sowie der parallele mechanische Dispergierprozess ist dabei umso kürzer, je höher die gewählte Basenkonzentration ist. Als Base wird vorzugsweise Ammoniak in Form einer 1 bis 12%igen wässrigen Lösung verwendet, andere Basen, wie z.B. NaOH, Diäthylamin oder KOH, können grundsätzlich auch eingesetzt werden. Die Volumenverhältnisse Base zu Sol liegen üblicherweise zwischen 1:2 und 1:4, Infolge der äußerst rasch verlaufenden Gelierungsreaktion wird der gesamte Partikel-Herstellungsprozess einschließlich der Einkapselung der Lumineszenzmarker innerhalb von einer Stunde bewerkstelligt. Dies stellt gegenüber alien herkömmlichen Verfahren zu Herstellung lumineszierender Partikel eine Zeitersparnis von bis zu 90 % dar.

Dem Herstellungsprozess schließen sich mehrere Waschungen mit Alkohol und Wasser an. Die erhaltenen Magnetträger werden üblicherweise in Wasser aufbewahrt. Danach können die gewonnenen Silicagel-Partikel direkt zur weiteren Funktionalisierung herangezogen werden.

Zur Ankopplung der Lumineszenz Partikel an die entsprechenden Biomoleküle wie Proteine, Antikörper, Peptide, Enzyme, Nukleinsäuren, Oligosaccharide, die durchweg als Zielsubstanzen, Rezeptoren, Bioliganden oder Analyt-Sonden fungieren, werden die hinlänglich bekannten Aktivierungs- und Kopplungsverfahren für Silicagel- bzw. silanisierte Träger verwand. Dazu gehören die Umsetzungen mit funktionellen Alkoxysilanen, die z. B. über Amino-, Epoxy-, Mercapto-, Isothiocyanat-, Acryl-ader Halogen-Gruppen verfügen. Beispiele für solche Aktivierungs-bzw. Kopplungsagenzien sind: 3-Aminopropyl-triethoxysilan, 3-Aminopropyltrimethoxysilan, 3-Glycidyloxypropyltrimethoxysilan, 3-Glycidyloxypropylmethyl-diethoxysilan, 3-Mercaptopropyl-trimethoxysilan, 3-Isothiocyanatopropyl-triethoxysilan, Methacryloxypropyltriethoxysilan, Chlor-propyl-triethoxysilan. Auch die Einführung von Carboxyl-, Hydroxyl oder Aldehyd-Gruppen Ober eine entsprechende Derivatisierung der obigen Alkoxysilane z. B. mit Aminocarbonsäuren, Glutardialdehyd oder Hydrolyse der Epoxy-Gruppen mit Säuren oder Basen zum Hydroxy-Derlvat ist allgemein aus dem Stand der Technik bekannt. Ebenso führt die Umsetzung der Epoxy-aktivierten Träger u.a. mit Carbonsäuren, Sulfiten, Thiosulfaten, Amino-Substituierten Carbonsäuren wie z. B. Nitrilotriessigsäure oder Iminodiessigsäure nach den bekannten Verfahren zu einem Metallchelat-Träger, Auch die Aktivierung der Silicagel-Partikel über photoaktive, beispielsweise Arylazid- oder Diazirin Funktionen-tragende Agenzien, die durch UV-Bestrahlung zunächst an den Träger gekoppelt und anschliessend mit dem Bioliganden bzw. dem Biomolekül abreagieren können, sind ebenfalls in einfacher Weise durchführbar. Substanzen dieser Art sind z. B.: N-Hydroxysuccinimidyl-4-azidobenzoat, [2-Nitro-4-[3-(trifluormethyl)-3H-diazirin-3-y1]phenoxy]acetyl-N-hydroxysuccinimidat, N-Hydroxysuccinimidyl-(4-azidophenyl)-1, 3'-dithioproplonat, N-[m-[3-(tri-fluormethyl)diazirin-3-yl]phenyl]-4-maleimidobutyramid.

Auf eine detaillierte Beschreibung der verschiedenen Aktivierungen, Funktionalisierungen sowie Kopplungen kann an dieser Stelle verzichtet werden, da die speziellen Reaktionsmethoden allgemein bekannt sind und von einem Fachmann auf diesem Gebiet jeder Zeit genutzt werden können (vgl. Vansant et al., in: "Characterization and Chemical Modification of the Silica Surface, Hrsg. Delmon und Yates, Elsevier, Amsterdam, 1997 ; Scientific and Clinical Applications of Magnetic Carriers", Häfeli et al. (Hrsg.), Plenum Press, New York, 1997; Shriver-Lake in:"Immobilized Biomolecules in Analysis", Cass und Ligler Hrsg., Oxford University Press, 1998; WO 99/01766; Lottspeich und Zorbas Hrsg., in:"Bioanalytik", Spektrum Verlag, Heidelberg, 1998). Die erwähnten prinzipiellen Ausführungsformen sind daher in keiner Weise als limitierende Offenbarungen aufzufassen.

Die Anwendung der erfindungsgemässen Lumineszenz-Partikel erstreckt sich auf sämtliche Gebiete der Bioanalytik und Diagnostik, die zum Nachweis oder Quantifizierung spezifischer Substanzen bzw. Analyten eine Farbreaktion, die Lumineszenz oder Radioaktivität nutzen. Beispiele hierfür sind der Immunoassay in Form des Radio-oder des Enzymlmmunoassays, der Nukleinsäurenschwais, die Proteomanalyse, die DNA-Sequenzierung, der Phage-Display, die Zellmarkierung, der Nukleinsäure-Array oder die Zellmarkierung. Ferner lassen sich die partikulären Lumineszenzmarker in der Durchflußcytometrie bzw. im fluoreszenzaktivierten Zellsorter (fluoresceneactivated cell sorter, FACS®) oder beim Durchtesten von DNA-oder Protein-Bibliotheken einsetzen.

In den nachfolgenden Beispielen werden die erfindungsgemässen Produkte und Verfahren näher beschrieben, ohne diese zu begrenzen.

### Beispiel 1

5 ml Tetramethoxysilan werden zusammen mit 2 ml 0,05 M HC1 in einem Ultraschallbad für 10 Minuten bei Raumtemperatur beschallt. 2 ml des gewonnenen klaren Sols werden mit 1 ml 0,05 %iger Rhodamin B-Lösung versetzt. Die erhaltene Mischung wird in 25 ml 0,4 ml Korantin (BASF) enthaltendem Hexan eingetragen, Der Ansatz wird mit einem Dispergierwerkzeug (Ultra-Turrax) für 3 Sekunden bei 20.000 U/min. dispergiert. Nach erfolgter Zugabe von 1 ml 1%ige Ammoniak Lösung wird noch 5 Sekunden weiter dispergiert. Nach weiteren 5 Minuten werden die Teilchen mittels zweiminütiger Zentrifugation sedimentiert. Der Überstand wird abdekantiert und je dreimal mit je ca. 10 ml Ethanol, Aceton und Wasser nachgewaschen. Es werden Lumineszenzpartikel mit einer Teilchengrösse von 1-3 im gewonnen.

Die gewonnen Teilchen werden anschließend mehrfach mit wasserfreiem Toluol gewaschen und anschließend unter Argonatmosphäre mit 5 ml wasserfreiem Toluol und 2 ml 3-GlycidyloXypropyl-trimethoxysilan 3 Stunden bei 90 °C unter Rühren umgesetzt. Danach wird fünfmal mit Toluol und Aceton nachgewaschen.

100 mg des gewonnenen Produktes werden sodann dreimal mit 0,5 molarem Phosphat-Puffer, pH 8,5, gewaschen und anschließend mit 2 ml desselben Puffers, in dem 10 mg Streptavidin gelöst sind, bei 40 °C 5 Stunden inkubiert. Das Produkt wird anschliessend fünfmal unter Anwendung eines jeweiligen Zentrifugationsschrittes mit 0,05 M Phosphat-Puffer, pH 7,2 gewaschen. Zur Blockierung restlicher Epoxy-Gruppen wird das gekoppelte Produkt 24 Stunden in 1 %iger Äthanolamln-Lösung, die 0,1% Serum Albumin enthält, bei Raumtemperatur aufbewahrt. Danach erfolgt mehrfaches Waschen mit 0,05 Tris/HC1-Puffer, pH 8,0.

Es resultiert ein Produkt, das zur Bindung bzw. Markierung biotinylierter Biomoleküle eingesetzt werden kann.

### Beispiel 2

2 ml des analog Beispiel 1 hergestellten Silica-Sols, werden mit 10 mg nach einer Vorschrift von Sookial et al. (Adv. Mater., Vol. 10, 1083, 1998) synthetisierten CdS-Halbleiter-Nanokristallen, die eine mittlere Teilchengrösse von 138 nm aufweisen, vermischt und anschließend 2 Min. bei Raumtemperatur beschallt. Die Mischung wird in 25 ml Toluol, das je 2,5 Vol% Span 60 und 0,5 Vol% Tween 80 gelöst enthält, mit Hilfe eines Ultra-Turrax bei 20.000 U/min. 5 Sekunden dispergiert, Nach Zugabe von 1 ml 6%iger Ammoniak-Lösung wird noch 5 Sekunden weiter dispergiert. Es folgt die Separation und Aufarbeitung der Partikel analog Beispiel 1. Es werden Lumineszenzpartikel mit einer mittleren Teilchengrösse von 3,6 im gewonnen, deren Emissionsmaximum bei 510 nm liegt.

Zur Aktivierung der Teilchen werden 75 mg Lumineszenzpartikel in Gegenwart von [2-Nitro-4-[3- (trifluormethyl)-3H- diazirin-3-yl]phenoxy]acetyl-N-hydroxysuccinimidat (Ansatz: 5 mg gelöst in 0,5 ml Ethanol-Wasser (1:1)), mit Hilfe des Stratalinker UV 2400 (Stratagene) für 20 Minuten bestrahlt.

An das aktivierte Produkt können nach entsprechendem Waschvorgang mit Äthanol und Wasser Aminogruppen-haltige Biomoleküle, Liganden oder Rezeptoren wie Nukleinsäuren, Proteine oder Antikörper nach den bekannten Methoden (Matson und Little, J.Chromatogr., Vol. 458,67, 1988) gekoppelt werden.

### Beispiel 3

0,5 ml Tetraethoxysilan werden mit 0,1 ml Wasser und 0,08 ml 0,1 M HC1 vermischt und 10 Minuten bei Raumtemperatur in einem Ultraschallbad beschallt. 0,2 ml des gewonnenen Sols werden mit 5 mg (YYbEr)₂0₂S, das nach einer Vorschrift von Hampl et al. (Anal. Biochem., Vol. 288, 176, 2001) hergestellt wurde, vermischt und 5 Minuten im Ultraschallbad behandelt. Der Mischung werden sodann 30 mg Magnetit-Pulver (Bayferrox 318M, Fa. Bayer, FRG) zugesetzt. Die Mischung wird nochmals für 2 Minuten beschallt. Die Mischung wird anschliessend unter Rühren (Ultra-Turrax) bei 12.000 U/min in 3 ml Trichlorethylen, in dem 2 Vol% Dehymuls HRE7® und 0,5 Vol% Prisorine3700® gelöst sind, dispergiert. Während des Dispergiervorganges werden 0,08 ml 6%ige wässrige Ammoniaklösung zugefügt. Es wird noch weitere 5 Sekunden weitergerührt. Separation und Aufarbeitung der gewonnenen Lumineszenzpartikel erfolgt analog Beispiel 1.

Man erhält Lumineszenzparükel mit einer mittleren Größe von 6,7 im.

Das 3 Stunden im Vakuum getrocknete Produkt wird fünfmal mit getrocknetem Toluol nach jeweiliger magnetischer Abtrennung gewaschen und anschließend 12 Std. nach Zugabe von 3 ml Toluol und 0,25 ml 3-Aminopropyltriäthoxysilan am Rückfluss gekocht. Die Magnetpartikel werden wieder magnetisch abgetrennt und mit ca. 5 ml Toluol und Chloroform 3-mal gewaschen. Es folgt eine mehrstündige Trocknung im Vakuum. Das aminomodifizierte Produkt wird anschließend mit 6%iger Glutaraldehyd-Lösung in 4 ml 0,1 M Na-Carbonat-Puffer, pH 9.0, für 2 Std. Bei 35°C umgesetzt. Es wird anschließend mit 0,1 M Phosphat-Puffer, pH 7.2, Intensiv nachgewaschen.

An die gewonnenen Aldehyd-funktionalisierten Lumineszenzpartikel können anschliessend nach den bekannten Verfahren Aminogruppen-tragende Biomoleküle wie Proteine, Peptide oder em 5'-Ende mit Aminogruppensubstituierte Nukleinsäuren oder Oligonucleotid nach den bekannten Verfahren gekoppelt werden. Die so funktionalisierten Lumineszenzpartikel lassen sich als Sonden in Protein-oder Nukleinsäure-Arrays einsetzen.

## Patentansprüche

1. Sphärische, lumineszierende Silicagel-Partikel, die in einer transparenten Silicagel-Matrix eine oder mehrere lumineszierende Substanzen enthalten,
wobei die Silicagel-Matrix erhältlich ist, indem eine Mischung bestehend aus einer verdünnten Säure und Alkoxysilanen zu einem klaren Silica-Sol kondensiert wird;
das klare Silica-Sol mit einem oder mehreren lumineszierenden Substanzen homogen vermischt wird;
die Sol-Lumineszenz-Substanz-Mischung in einer mit Wasser nicht mischbaren organischen Phase dispergiert und die Sol-Lumineszenz-Substanz-Mischung während oder nach dem Dispergiervorgang durch Zugabe einer Base vernetzt wird,
und
wobei die Silicagel-Matrix funktionelle Gruppen aufweist, die mit Biomolekülen koppelbar sind.

2. Sphärische, Iumineszierende Silicagel-Partikel nach Anspruch 1, **dadurch gekennzeichnet, dass** sie zusätzlich ein magnetisches Kolloid enthalten.

3. Sphärische, lumineszierende Silicagel-Partikel nach einem der Ansprüche 1 oder 2, **dadurch gekennzeichnet, dass** die lumineszierende(n) Substanz(en) aus der Gruppe, die aus lumineszierenden Marker, Haibleiter-Nanokristalle, Halbleiter aus der Gruppe IV/A, up-converting phosphore und der lumineszierenden Proteinen besteht, ausgewählt ist/sind.

4. Sphärische, lumineszierende Silicagel-Partikel nach einem der Ansprüche 1 bis 3, **dadurch gekennzeichnet, dass** die Partikelgröße 0,5 bis 50 µm beträgt.

5. Sphärische, lumineszierende Silicagel-Partikel nach einem der Ansprüche 1 bis 4, **dadurch gekennzeichnet, dass** sie keine Eigenfluoreszenz aufweisen,

6. Sphärische, lumineszierende Silicagel-Partikel nach einem der Ansprüche 1 bis 5, **dadurch gekennzeichnet, dass** die genannte(n) Iumineszierende(n) Substanz(en) in die Silicagel-Partikel eingekapselt sind.

7. Sphärische, lumineszierende Silicagel-Partikel nach einem der Ansprüche 1 bis 6, **dadurch gekennzeichnet, dass** die lumineszierende(n) Substanz(en) aus der Gruppe der Substanzen ausgewählt ist/sind, die eine Fluoreszenz, Phosphoreszenz, Chemilumineszenz, Elektrolumineszenz oder einen Lumineszenz-Energietransfer aufweisen.

8. Sphärische, lumineszierende Silicagel-Partikel nach einem der Ansprüche 1 bis 7, **dadurch gekennzeichnet, dass** die Konzentration der lumineszierenden Substanzen 1 bis 10 Gew.-% beträgt.

9. Sphärische, lumineszierende Silicagel-Partikel nach einem der Ansprüche 1 bis 8, **dadurch gekennzeichnet, dass** die lumineszierenden Substanzen unterschiedliche Emissionsfrequenzen aufweisen.

10. Sphärische, lumineszierende Silicagel-Partikel nach einem der Ansprüche 1 bis 9, **dadurch gekennzeichnet, dass** die Iumineszierenden Substanzen Moleküle sind, deren Anregungsfrequenz höher ist als die Emissionsfrequenz.

11. Sphärisch, lumineszierende Silicagel-Partikel nach einem der Ansprüche 1 bis 10, **dadurch gekennzeichnet, dass** die lumineszierenden Substanzen aus Halbleiter-Nanokristallen bestehen, die aus Elementen der Gruppe IIIA und VA, Gruppe IIB und VIA oder Gruppe IVA des Periodensystems gebildet sind.

12. Sphärische, lumineszierende Silicagel-Partikel nach einem der Ansprüche 1 bis 11, **dadurch gekennzeichnet, dass** die lumineszierenden Halbleiter-Nanokristalle mit Kupfer- und/oder Silberzusätzen dotiert sind.

13. Sphärische, lumineszierende Silicagel-Partikel nach einem der Ansprüche 1 bis 9, **dadurch gekennzeichnet, dass** die lumineszierenden Substanzen Anregungsfrequenzen aufweisen, die niedriger als die Emissionsfrequenzen sind.

14. Sphärische, lumineszierende Silicagel-Partikel nach einem der Ansprüche 1 bis 9 und 13, **dadurch gekennzeichnet, dass** die lumineszierenden Substanzen mikrokristalline Verbindungen aus Seltenen Erden und/oder Yttrium mit Elementen aus der Gruppe VIA und/oder VIIA des Periodensystems sind.

15. Sphärische, lumineszierende Silicagel-Partikel nach einem der Ansprüche 1 bis 14, **dadurch gekennzeichnet, dass** die lumineszierenden Substanzen Metall-Chelatverbindungen sind, deren Zentraiatom aus der Gruppe VIII, IB, IIB des Periodensystems oder der Gruppe der Seltenen Erden ausgewählt ist.

16. Sphärische, Iumineszierende Silicagel-Partikel nach einem der Ansprüche 1 bis 15, **dadurch gekennzeichnet, dass** die lumineszierenden Substanzen Pyrrolfarbstoffe sind.

17. Sphärische, lumineszierende Silicagel-Partikel nach einem der Ansprüche 1 bis 15, **dadurch gekennzeichnet, dass** die lumineszierenden Substanzen lumineszierende Proteine sind.

18. Sphärische, lumineszierende Silicagel-Partikel nach einem der Ansprüche 1 bis 17, **dadurch gekennzeichnet, dass** die Sillcagel-Partikel zusätzlich ein magnetisches Kolloid enthalten oder mit eingekapselt ist.

19. Sphärische, lumineszierende Silicagel-Partlkel nach einem der Ansprüche 1 bis 18, **dadurch gekennzeichnet, dass** das magnetische Kolloid aus der Gruppe ausgewählt ist, die ferro-, ferri- und superparamagnetische Verbindungen und Ferrofluide umfasst.

20. Sphärische, lumineszierende Silicagel-Partikel nach einem der Ansprüche 1 bis 19, **dadurch gekennzeichnet, dass** das magnetische Kolloid in einer Konzentration von 10 bis 50 Gew-%, bezogen auf das Silicagel-Partikel, vorliegt.

21. Sphärische, lumineszierende Silicagel-Partikel nach einem der Ansprüche 1 bis 20, **dadurch gekennzeichnet, dass** die Silicagele funktionelle Gruppen aufweisen, die mit Biomolekülen, ausgewählt aus der Proteine, Peptide, Zellrezeptoren, Nukleinsäuren, Nukleinsäure-Fragmente, Polysaccharide, Oligosaccharide, Antikörper, Antikörper-Fragmente, Streptavidin, Avidin, Biotin und Enzyme umfassenden Gruppe, koppelbar sind, oder an die eine oder mehrere solcher Biomoleküle gekoppelt sind.

22. Verfahren zur Herstellung sphärischer, lumineszierender Silicagel-Partikel, nach den Ansprüchen 1 bis 21, **dadurch gekennzeichnet, dass**
eine Mischung bestehend aus einer verdünnten Säure und Alkoxysilanen zu einem klaren Silica-Sol kondensiert wird;
das klare Silica-Sol mit einem oder mehreren lumineszierenden Substanzen homogen vermischt wird;
die Sol-Lumineszenz-Substanz-Mischung in einer mit Wasser nicht mischbaren organischen Phase dispergiert und
die Sol-Lumineszenz-Substanz-Mischung während oder nach dem Dispergiervorgang durch Zugabe einer Base vernetzt wird.

23. Verfahren zur Herstellung lumineszierender, transparenter Silicagel-Partikel gemäß Anspruch 22, **dadurch gekennzeichnet, dass** der Sol-Lumineszenz-Substanz-Mischung 10 bis 50 Gew.-% einer ferro-, ferri- oder superparamagnetischen Substanz zugesetzt werden.

24. Verfahren zur Herstellung lumineszlerender Silicagel-Partikel gemäß Anspruch 22 oder 23, **dadurch gekennzeichnet, dass** die mit Wasser nicht mischbare organische Phase eine oder mehrere oberflächenaktive Substanz(en) in einer Konzentration von 0,1 bis 15 Vol.-% enthält.

25. Verfahren zur Herstellung lumineszierender Silicagel-Partikel gemäß einem der Ansprüche 22 bis 24, **dadurch gekennzeichnet, dass** das Volumenverhältnis Sol zu organischer Phase 1:5 bis 1:30 beträgt.

26. Verfahren zur Herstellung lumineszierender Silicagel-Partikel gemäß einem der Ansprüche 22 bis 25, **dadurch gekennzeichnet, dass** der Dispersions-Vernetzungsvorgang 2 bis 30 Sekunden dauert.

27. Verfahren zur Herstellung lumineszierender Sillcagel-Partikel gemäß einem der Ansprüche 22 bis 26, **dadurch gekennzeichnet, dass** dem Sol vor dem Dispersionsvorgang 1-20 Vol.-% einer wässrigen Lösung eines organischen Polymeren, Polysaccharids oder Proteins zugemlscht werden.

28. Verwendung von sphärischen, lumineszierenden Silicagel-Partikel, nach den Ansprüchen 1 bis 21,
für die Analyse und/oder Diagnostik von Nukleinsäuren, Nukleinsäure-Fragmenten, Proteinen, Peptiden, Antikörpern, Antikörper-Fragmenten, Zellen, Zellrezeptoren, biotinylisierten Biomolekülen sowie für die Durchtestung von Protein- oder Nukleinsäure-Bibliotheken, als Sonden im Rahmen der Array-Technologie oder für die Nukleinsäure-Sequenzierung.

## Claims

1. Spherical, luminescent silica gel particles which contain one or more luminescent substances in a transparent silica gel matrix, wherein the silica gel matrix is obtained by
condensing a mixture consisting of a diluted acid and alkoxysilanes to a clear silica sol;
homogeneously mixing the clear silica sol with one or more luminescent substances;
dispersing the sol-luminescences substance mixture in an organic phase that is not miscible with water and
cross-linking the sol-luminescence substance mixture during or after dispersion by adding a base,
and
wherein the silica gel matrix has functional groups which can be coupled with biomolecules.

2. Spherical, luminescent silica gel particles according to Claim 1, **characterized in that** they additionally contain a magnetic colloid.

3. Spherical, luminescent silica gel particles according to Claim 1 or 2, **characterized in that** the luminescent substance(s) is/are selected from the group consisting of luminescent markers, semiconductor nanocrystals, semiconductors from the Group IVA, up-converting phosphors and luminescent proteins.

4. Spherical, luminescent silica gel particles according to one of Claims 1 to 3, **characterized in that** the particle size is 0.5 to 50 µm.

5. Spherical, luminescent silica gel particles according to one of Claims 1 to 4, **characterized in that** they are non-autofluorescent.

6. Spherical, luminescent silica gel particles according to one of Claims 1 to 5, **characterized in that** the said luminescent substance(s) is/are encapsulated in the silica gel particles.

7. Spherical, luminescent silica gel particles according to one of Claims 1 to 6, **characterized in that** the luminescent substance(s) is/are selected from the group of substances which display fluorescence, phosphorescence, chemoluminescence, electroluminescence or a luminescence energy transfer.

8. Spherical, luminescent silica gel particles according to one of Claims 1 to 7, **characterized in that** the concentration of the luminsecent substances is 1 to 10%-wt.

9. Spherical, luminescent silica gel particles according to one of Claims 1 to 8, **characterized in that** the luminescent substances display different emission frequencies.

10. Spherical, luminescent silica gel particles according to one of Claims 1 to 9, **characterized in that** the luminescent substances are molecules whose excitation frequency is higher than the emission frequency.

11. Spherical, luminescent silica gel particles according to one of Claims 1 to 10, **characterized in that** the luminescent substances consist of semiconductor nanocrystals formed from elements of the Group IIIA and VA, Group IIB and VIA or Group IVA of the periodic table of elements.

12. Spherical, luminescent silica gel particles according to one of Claims 1 to 11, **characterized in that** the luminescent semiconductor nanocrystals are doped with copper and/or silver additives.

13. Spherical, luminescent silica gel particles according to one of Claims 1 to 9, **characterized in that** the luminesecent substances have excitation frequencies that are lower than the emission frequencies.

14. Spherical, luminescent silica gel particles according to one of Claims 1 to 9 and Claim 13, **characterized in that** the luminescent substances are microcrystalline compounds of rare earths and/or yttrium with elements from the Group VIA and/or VIIA of the periodic table of elements.

15. Spherical, luminescent silica gel particles according to one of Claims 1 to 14, **characterized in that** the luminescent substances are metal-chelate compounds whose central atom has been chosen from the Group VIII, IB, IIB of the periodic table of elements or of the group of rare earths.

16. Spherical, luminescent silica gel particles according to one of Claims 1 to 15, **characterized in that** the luminescent substances are pyrrole dyes.

17. Spherical, luminescent silica gel particles according to one of Claims 1 to 15, **characterized in that** the luminescent substances are luminescent proteins.

18. Spherical, luminescent silica gel particles according to one of Claims 1 to 17, **characterized in that,** in addition, a magnetic colloid is contained or has been encapsulated in the silica gel particle.

19. Spherical, luminescent silica gel particles according to one of Claims 1 to 18, **characterized in that** the magnetic colloid is selected from the group comprising ferro-, ferri- and superparamagnetic compounds and ferrofluids.

20. Spherical, luminescent silica gel particles according to one of Claims 1 to 19, **characterized in that** the magnetic colloid is present in a concentration of 10-50% by weight relative to the silica gel particle.

21. Spherical, luminescent silica gel particles according to one of Claims 1 to 20, **characterized in that** the silica gels have functional groups that can be coupled to biomolecules, selected from the group comprising proteins, peptides, cell receptors, nucleic acids, nucleic acid fragments, polysaccharides, oligosaccharides, antibodies, antibody fragments, streptavidin, avidin, biotin, and enzymes, or that are coupled to one or more of such biomolecules.

22. Process for the production of spherical, luminescent silica gel particles according to one of Claims 1 to 21, **characterized in that**
a mixture consisting of a diluted acid and alkoxysilanes is condensed to a clear silica sol;
the clear silica sol is homogeneously mixed with one or more luminescent substances;
the sol-luminescences substance mixture is dispersed in an organic phase that is not miscible with water and
the sol-luminescence substance mixture is cross-linked during or after dispersion by adding a base.

23. Process for the production of luminescent, transparent silica gel particles according to Claim 22, **characterized in that** 10-50% by weight of a ferro-, ferri- or superparamagnetic substace is added to the sol-luminescence subance mixture.

24. Process for the production of luminescent silica gel particles according to Claim 22 or 23, **characterized in that** the organic phase that is not miscible with water contains one or more surfactive substances in a concentration of 0.1 to 15% by volume.

25. Process for the production of luminescent silica gel particles according to one of Claims 22 to 24, **characterized in that** the volume ratio of sol to organic phase is 1:5 to 1:30.

26. Process for the production of luminescent silica gel particles according to one of Claims 22 to 25, **characterized in that** the dispersion-cross-linking process takes 2 to 30 seconds.

27. Process for the production of luminescent silica gel particles according to one of Claims 22 to 26, **characterized in that** 1 - 20% by volume of an aqueous solution of an organic polymer, polysaccharide or protein is mixed with the sol before dispersion.

28. Use of spherical, luminescent silica gel particles according to one of Claims 1 to 21 for the analysis and/or diagnostic of nucleic acids, nucleic acid fragments, proteins, peptides, antibodies, antibody fragments, cells, cell receptors, biotinylated biomolecules and for testing protein or nucleic acid libraries, as sensors in the context of array technology or for nucleic acid sequencing.

## Revendications

1. Particules de silicagel sphériques et luminescentes qui contiennent une ou plusieurs substances luminescentes dans une matrice de silicagel transparente,
la matrice de silicagel étant obtenue en condensant un mélange constitué d'un acide dilué et d'alcoxysilanes pour donner un sol de silice clair ;
le sol de silice clair étant mélangé de façon homogène à une ou plusieurs substances luminescentes ;
le mélange sol/substance luminescente étant dispersé dans une phase organique non miscible avec l'eau et
le mélange sol/substance luminescente étant réticulé par apport d'une base pendant ou après le processus de dispersion,
et
la matrice de silicagel comportant des groupes fonctionnels qui peuvent être couplés à des biomolécules.

2. Particules de silicagel sphériques et luminescentes selon la revendication 1, **caractérisées en ce qu'**elles contiennent en plus un colloïde magnétique.

3. Particules de silicagel sphériques et luminescentes selon l'une des revendications 1 ou 2, **caractérisées en ce que** la ou les substances luminescentes est/sont choisie(s) dans le groupe qui est constitué de marqueurs luminescents, de nanocristaux semi-conducteurs, de semi-conducteurs du groupe IV/A, de phosphores élévateurs et des protéines luminescentes.

4. Particules de silicagel sphériques et luminescentes selon l'une des revendications 1 à 3, **caractérisées en ce que** la taille des particules va de 0,5 à 50 µm.

5. Particules de silicagel sphériques et luminescentes selon l'une des revendications 1 à 4, **caractérisées en ce qu'**elles ne présentent aucune fluorescence propre.

6. Particules de silicagel sphériques et luminescentes selon l'une des revendications 1 à 5, **caractérisées en ce que** ladite ou lesdites substances luminescentes sont encapsulées dans les particules de silicagel.

7. Particules de silicagel sphériques et luminescentes selon l'une des revendications 1 à 6, **caractérisées en ce que** la ou les substances luminescentes est/sont choisie(s) dans le groupe des substances qui présentent une fluorescence, une phosphorescence, une chimiluminescence, une électroluminescence ou un transfert d'énergie par luminescence.

8. Particules de silicagel sphériques et luminescentes selon l'une des revendications 1 à 7, **caractérisées en ce que** la concentration en substances luminescentes va de 1 à 10% en poids.

9. Particules de silicagel sphériques et luminescentes selon l'une des revendications 1 à 8, **caractérisées en ce que** les substances luminescentes présentent différentes fréquences d'émission.

10. Particules de silicagel sphériques et luminescentes selon l'une des revendications 1 à 9, **caractérisées en ce que** les substances luminescentes sont des molécules dont la fréquence d'excitation est supérieure à la fréquence d'émission.

11. Particules de silicagel sphériques et luminescentes selon l'une des revendications 1 à 10, **caractérisées en ce que** les substances luminescentes sont constituées de nanocristaux semi-conducteurs qui sont formés à partir d'éléments du groupe IIIA et VA, du groupe IIB et VIA ou du groupe IVA du système périodique.

12. Particules de silicagel sphériques et luminescentes selon l'une des revendications 1 à 11, **caractérisées en ce que** les nanocristaux semi-conducteurs luminescents sont dopés par ajouts de cuivre et/ou d'argent.

13. Particules de silicagel sphériques et luminescentes selon l'une des revendications 1 à 9, **caractérisées en ce que** les substances luminescentes présentent des fréquences d'excitation qui sont inférieures aux fréquences d'émission.

14. Particules de silicagel sphériques et luminescentes selon l'une des revendications 1 à 9 et 13, **caractérisées en ce que** les substances luminescentes sont des composés microcristallins constitués de terres rares et/ou d'yttrium avec des éléments du groupe VIA et/ou VIIA du système périodique.

15. Particules de silicagel sphériques et luminescentes selon l'une des revendications 1 à 14, **caractérisées en ce que** les substances luminescentes sont des composés métalliques chélatés dont l'atome central est choisi dans le groupe VIII, IB, IIB du système périodique ou le groupe des terres rares.

16. Particules de silicagel sphériques et luminescentes selon l'une des revendications 1 à 15, **caractérisées en ce que** les substances luminescentes sont des colorants pyrroliques.

17. Particules de silicagel sphériques et luminescentes selon l'une des revendications 1 à 15, **caractérisées en ce que** les substances luminescentes sont des protéines luminescentes.

18. Particules de silicagel sphériques et luminescentes selon l'une des revendications 1 à 17, **caractérisées en ce que** les particules de silicagel contiennent en plus un colloïde magnétique ou sont encapsulées avec celui-ci.

19. Particules de silicagel sphériques et luminescentes selon l'une des revendications 1 à 18, **caractérisées en ce que** le colloïde magnétique est choisi dans le groupe qui comporte des composés ferromagnétiques, ferrimagnétiques et super-paramagnétiques et des ferro-fluides.

20. Particules de silicagel sphériques et luminescentes selon l'une des revendications 1 à 19, **caractérisées en ce que** le colloïde magnétique est à une concentration de 10 à 50% en poids par rapport à la particule de silicagel.

21. Particules de silicagel sphériques et luminescentes selon l'une des revendications 1 à 20, **caractérisées en ce que** les silicagels comportent des groupes fonctionnels qui peuvent être couplés à des biomolécules choisies dans le groupe comportant des protéines, des peptides, des récepteurs cellulaires, des acides nucléiques, des fragments d'acides nucléiques, des polysaccharides, des oligosaccharides, des anticorps, des fragments d'anticorps, la streptavidine, l'avidine, la biotine et des enzymes, ou qui sont couplés à une ou plusieurs de ces biomolécules.

22. Procédé de fabrication de particules de silicagel sphériques et luminescentes selon les revendications 1 à 21, **caractérisé en ce que**
un mélange constitué d'un acide dilué et d'alcoxysilanes est condensé pour donner un sol de silice clair;
le sol de silice clair est mélangé de façon homogène à une ou plusieurs substances luminescentes ;
le mélange sol/substance luminescente est dispersé dans une phase organique non miscible à l'eau et
le mélange sol/substance luminescente est réticulé par apport d'une base pendant ou après le processus de dispersion.

23. Procédé de fabrication de particules de silicagel luminescentes et transparentes selon la revendication 22, **caractérisé en ce que** le mélange sol/substance luminescente est additionné de 10 à 50% en poids d'une substance ferromagnétique, ferrimagnétique ou super-paramagnétique.

24. Procédé de fabrication de particules de silicagel luminescentes selon la revendication 22 ou 23, **caractérisé en ce que** la phase organique non miscible à l'eau contient une ou plusieurs substances tensioactives à une concentration de 0,1 à 15% en volume.

25. Procédé de fabrication de particules de silicagel luminescentes selon l'une des revendications 22 à 24, **caractérisé en ce que** le rapport en volume sol sur phase organique est de 1:5 à 1:30.

26. Procédé de fabrication de particules de silicagel luminescentes selon l'une des revendications 22 à 25, **caractérisé en ce que** le processus de réticulation et de dispersion dure 2 à 30 secondes.

27. Procédé de fabrication de particules de silicagel luminescentes selon l'une des revendications 22 à 26, **caractérisé en ce que** l'on ajoute avec mélange au sol, avant le processus de dispersion, 1 à 20% en volume d'une solution aqueuse d'un polymère organique, d'un polysaccharide ou d'une protéine.

28. Utilisation de particules de silicagel sphériques et luminescentes selon les revendications 1 à 21, comme sondes dans le cadre de la technologie des puces ou pour le séquençage d'acides nucléiques, dans l'analyse et/ou le diagnostique d'acides nucléiques, de fragments d'acides nucléiques, de protéines, de peptides, d'anticorps, de fragments d'anticorps, de cellules, de récepteurs cellulaires, de biomolécules biotinylées ainsi que pour le criblage de bibliothèques de protéines ou d'acides nucléiques.
